# EUROPEAN PATENT SPECIFICATION

(11) **EP 1 814 435 B1**
(45) Date of publication and mention of the grant of the patent: **19.10.2011**
(21) Application number: 05803207.9
(22) Date of filing: 11.11.2005
(51) Int. Cl.: A61B 1/31

(54) **SIGMOIDOSCOPE WITH OPTICAL COUPLING ELEMENT**
SIGMOIDOSKOP MIT OPTISCHEM KUPPLUNGSELEMENT
SIGMOIDOSCOPE AVEC ELEMENT DE COUPLAGE OPTIQUE

(30) Priority: 11.11.2004 AU 2004906484; 04.03.2005 AU 2005901048
(43) Date of publication of application: 08.08.2007
(73) Proprietor: Daltray Pty Ltd., Rose Bay, NSW 2029 (AU)
(72) Inventor: LUBOWSKI, David, Rose Bay, NSW 2029 (AU); MARSH, Peter, Crispin, Birchgrove, NSW 2041 (AU)
(74) Representative: Clegg, Richard Ian
(86) International application number: PCT/AU2005/001727
(87) International publication number: WO 2006/050574

(56) References cited:
- WO-A-00/06013
- WO-A-00/12000
- WO-A1-00/06013
- WO-A1-2004/098395
- WO-A2-97/14287
- GB-A- 2 354 447
- US-A- 4 569 333
- US-A- 4 852 551
- US-A- 5 792 045
- US-B1- 6 468 202

## Description

### FIELD OF THE INVENTION

This invention relates to medical instruments, and in particular to an instrument adapted for insertion into bodily cavities. The invention has been developed primarily as a sigmoidoscope for use in examination of the bowel cavity of a patient and for sigmoidoscopy generally.

### BACKGROUND OF THE INVENTION

The following description of the prior art is provided to place the invention in an appropriate technical context, and to enable the advantages flowing from it to be fully appreciated. References to the prior art should not, however, be interpreted as indicating that such art is well known, or forms part of common general knowledge, in the field.

It is frequently necessary for specialised medical practitioners to inspect the sigmoid colon or rectum of a patient. This procedure is commonly conducted with a rigid sigmoidoscope, which in the past typically consisted of a metal tube or speculum adapted at one end for insertion into the rectum of a patient, and adapted at the other end for connection with a manifold. The manifold was provided with an observation window, and a rubber insufflation bulb. The bulb was connectable via a spigot to the manifold for fluid communication with the interior of the speculum to enable pressurisation and insufflation of the bowel cavity. An illumination system was also provided with which at least a portion of the interior of the bowel interior could be illuminated during examination. In the past, after use and before reuse on a subsequent patient, the metal tube was sterilised. Subsequently, sigmoidoscopes were designed such that components coming into direct contact with the patient were adapted to be disposed of after use to avoid the time and expense of cleaning and sterilisation.

More recently, rigid sigmoidoscopes have employed a disposable speculum in the form of a hollow, light transmissive, plastic tube. The disposable speculum is typically purchased in a clean or sterile condition sealed in a bag together with a disposable obturator adapted for use with the speculum. In use, the disposable speculum is coupled to a non-disposable fibre-optic head incorporating a hinged window through which an obturator or biopsy instrument may be intermittently inserted and removed through the interior of the speculum.

In devices of this type, a light source is operatively coupled with the speculum via the fibre optic head so as to illuminate a circumferential end edge of the speculum, whereby light is directed through the wall of the speculum into the bowel cavity under examination. The fibre-optic head is also provided with a spigot for connection, via a flexible tube, to an insufflation bulb with which the bowel may be insufflated. After initial insertion of the sigmoidoscope, the obturator is withdrawn through the speculum.

Upon conclusion of an examination, the relatively inexpensive plastic speculum is disconnected from the fibre-optic head and disposed of The fibre-optic head together with the hinged window and the light source are relatively expensive components and are therefore not disposed of Rather, these components are retained for use with other specula in subsequent procedures. The insufflation bulb may be disconnected between uses but commonly remains connected to the fibre optic head.

This practice gives rise to the potential for cross-contamination and inflection from the reusable components of the sigmoidoscope, most notably the insufflation bulb and the fibre-optic head. The present applicant recognised this potential, which led to the invention of a new form of sigmoidoscope, as described in patent application number WO 00/06013 . That new form of sigmoidoscope addressed the problem of cross contamination via the insufflation bulb and the optical head. However, a problem remained in relation to the obturator, which has not hitherto been addressed by the applicant's earlier invention, or the prior art.

In use, an obturator consisting essentially of an elongate stem terminating in a domed head is typically inserted axially through the interior of the speculum until the domed head protrudes beyond the remote end of the speculum, so as temporarily to close off the insertion end of the speculum. This facilitates initial insertion of the speculum, following which the contaminated obturator is withdrawn, and typically disposed of. This withdrawal is necessary in order to provide an unobstructed viewing path through the interior of the speculum. However, a fundamental problem with known sigmoidoscope designs is that as the obturator is withdrawn, it can and in practice almost inevitably does inadvertently come into contact with non-disposable parts of the sigmoidoscope, or with disposable parts that subsequently contact non-disposable parts. This creates significant potential for cross-contamination.

A further problem is that when inserting the instrument or subsequently during the examination procedure, faecal matter may partially or completely occlude the open end of the speculum and hence obstruct the view. It is then necessary either to withdraw the instrument to clear the end, or reinsert the obturator or a cotton wool pledget to do so. This gives rise to further potential for contamination of the non-disposable components of the instrument.

In an attempt to address these problems, the present applicant's earlier patent application, as referenced above, disclosed the use of an integral, disposable obturator in the form of a sleeve around the main tube of the speculum. At the insertion end, the obturator sleeve incorporates a plurality of resiliently deformable petals or tangs adapted, in an extended configuration, to curve inwardly so as collectively to define a domed formation over the insertion end of the speculum. After insertion, the sleeve of the obturator is adapted to be telescopically withdrawn relative to the speculum. In this way, the petals or tangs resiliently retract around the speculum to a position behind the insertion end, to provide an unobstructed viewing path through the interior of the speculum.

While addressing the problem of the potential for initial contamination from the obturator, this arrangement is somewhat complex and expensive to manufacture and cannot readily be used to adapt existing sigmoidoscopes to a safer configuration.

Although these problems have been described in the context of sigmoidoscopy, it will be appreciated that similar difficulties may arise in analogous medical procedures.

It is an object of the present invention to provide an improved sigmoidoscope, which overcomes or ameliorates one or more of the disadvantages of the prior art, or at least provides a useful alternative.

WO 00/06013 discloses a sigmoidoscope according to the preamble of claim 1.

### DISCLOSURE OF THE INVENTION

The invention relates to a sigmoidoscope and a coupling element according to claim 1 and claim 20 respectively. Accordingly, in a first aspect, the invention provides a sigmoidoscope adapted for use in conjunction with a removable obturator to facilitate internal examination of the sigmoid colon of a patient, said sigmoidoscope including:
a generally tubular speculum having a sidewall defining a forward insertion end, a rearward observation end and a lumen, the lumen defining a substantially unobstructed viewing path between the observation end and the insertion end;
manually operable insufflation means in fluid communication with the lumen of the speculum, for insufflation of the sigmoid colon with an insufflation medium;
a coupling element having a proximal end for releasable connection to the observation end of the speculum, a distal end adapted for releasable connection to an optical head, and an optically transparent observation window effectively isolating the distal end from the proximal end;
the coupling element being adapted upon connection to substantially seal the observation end of the speculum and to preventing direct contact between the optical head and the speculum, without visually obstructing the viewing path.

Advantageously, it will be appreciated that this arrangement permits initial and intermittent insertion of the obturator through the speculum upon disconnection of the proximal end of the coupling element, while at all times isolating the optical head from direct contact with the speculum and the insufflation medium, so as to substantially eliminate the risk of contamination of the optical head and consequential cross-contamination of subsequent patients.

A "contaminant" as herein defined is any agent capable of infection and includes, without limitation, viruses, bacteria, fungi, protozoa, mycoplasma and organic or inorganic carriers of any of the above. "Contamination" carries the same sense of meaning.

The insufflation means preferably include a manually operable insufflation bulb or bellows and a gas conveying insufflation tube. The insufflation medium is preferably air.

In one arrangement, the insufflation tube is adapted for connection to a hollow spigot extending outwardly from the speculum. In this way, the insufflation tube and spigot are preferably adapted to establish direct fluid communication between the insufflation bulb or bellows and the interior region or lumen of the speculum.

In the embodiment according to the invention, the spigot extends outwardly from the coupling element, such that the insufflation medium is fed indirectly into the lumen of the speculum, via the coupling element. In this embodiment, the observation window is positioned intermediate the insufflation spigot and the distal end of the coupling element. Advantageously, this embodiment of the invention can be readily "retrofitted" for use in conjunction with conventional specula. It will be appreciated that the insufflation tube may be releasably connected to the speculum or the coupling element by any suitable means other than a spigot, and may also be formed integrally with the speculum or coupling element.

In one arrangement the speculum, insufflation means and coupling element are intended to be disposable, whereas the relatively expensive optical head and associated components are intended to be reusable, in the conventional sense in which these terms are generally understood by those skilled in the art of medical and surgical practice. In alternative arrangements, however, the insufflation means may also be reusable, subject to appropriate sterilisation procedures or contamination prevention means.

Such contamination prevention means may include, for example, a non-return valve, a filter such as a nanopore filter, an electrostatic precipitator or other means adapted to prevent internal surfaces from becoming contaminated either by contact with airborne contaminants via the insufflation medium, or by direct contact with a patient or other contaminated parts of the instrument. In this context, it will be understood that the term "insufflation means" includes any tubing, fluid conduits or other components communicating between the insufflation bulb or other source of insufflation medium, and the sigmoidoscope.

The optical head preferably incorporates an eyepiece to facilitate visual inspection of the colon through the observation window of the coupling element and the lumen of the speculum. The optical head preferably also includes connection means to enable releasable connection of a light source. In one arrangement the speculum and coupling element are formed from optically transparent or translucent plastics materials adapted to conduct light from the light source to the insertion end of the speculum, for illumination of the insufflated region of the colon during the examination procedure.

Preferably, the proximal end of the coupling element is adapted for releasable connection with the speculum by first connection means, which preferably take the form of a first twist-lock mechanism and associated sealing surfaces such that the connecting element effectively seals the lumen at the observation end of the speculum. Importantly, this arrangement provides an air-tight seal preventing the passage of any contaminated insufflation medium around or through the observation window to the distal side of the connecting element. In alternative arrangements however, the first connection means may take other forms, such as a threaded connection, a hinged connection, a tapered connection, a sliding connection, a magnetic connection or a press-fitted connection.

Preferably also, the distal end of the coupling element is adapted for releasable connection to the optical head by second connection means, which preferably take the form of complementary screws threads or second twist-lock mechanism. In alternative arrangements, however, it will be appreciated that other connection mechanisms such as hinged, tapered, sliding, magnetic or press-fitted connections may also be used.

The optical head preferably provides oblique connection of the light source, to provide an unobstructed axial viewing path through the optical head, the coupling element and the lumen. The optical head preferably includes fibre-optic elements to transmit light from the light source to the coupling element.

### BRIEF DESCRIPTION OF THE DRAWINGS

Preferred embodiments and arrangements of the invention will now be described, by way of example only, with reference to the accompanying drawings in which:-
Figure 1 is a side elevation view showing a sigmoidoscope, according to an arrangement ;
Figure 2 is a plan view of the sigmoidoscope of figure 1;
Figure 3 is a front end elevation of the sigmoidoscope of figures 1 and 2;
Figure 4 is an exploded perspective view of the sigmoidoscope of figures 1 to 3 showing the primary constituent components including the speculum, removable obturator, coupling element, optical head, light source and insufflation bellows;
Figure 5 is an enlarged perspective view of the coupling element;
Figure 6 is a side elevation view of the coupling element of figure 5;
Figure 7 is a cross-sectional view of the coupling element, taken along line 7-7 of figure 6;
Figure 8 is a side elevation view showing the sigmoidoscope with the optical head detached and a removable obturator installed, in an insertion configuration;
Figure 9 is a plan view of the sigmoidoscope of Figure 8 in the insertion configuration;
Figure 10 is a side elevation view showing a sigmoidoscope according to the embodiment of the invention, in which the insufflation tube is connected to a spigot formed on the coupling element;
Figure 11 is a plan view of the sigmoidoscope of figure 10;
Figure 12 is a front end elevation of the sigmoidoscope of figures 10 and 11;
Figure 13 is an enlarged perspective view of the coupling element from the sigmoidoscopes according to the embodiment of the invention, as shown in figures 10 to 12;
Figure 14 is a side elevation view of the coupling element of figure 13; and
Figure 15 is a cross-sectional view of the coupling element, taken along line 15-15 of figure 14.

### PREFERRED EMBODIMENT OF THE INVENTION AND ARRANGEMENTS

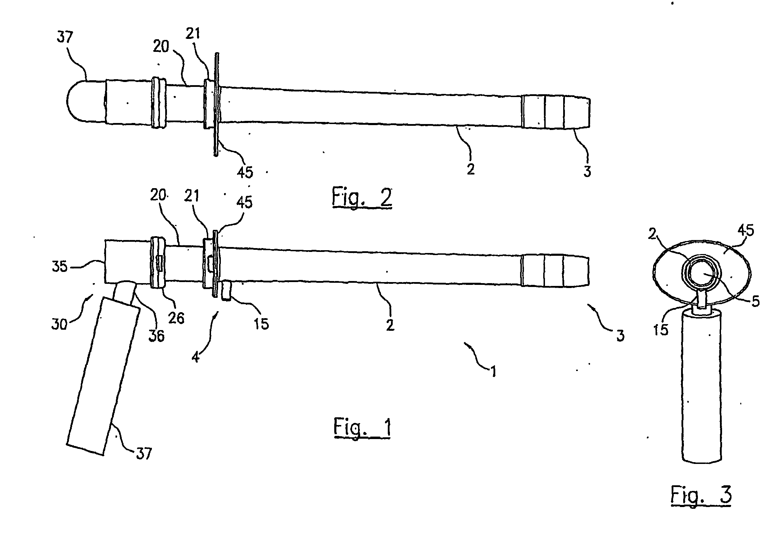

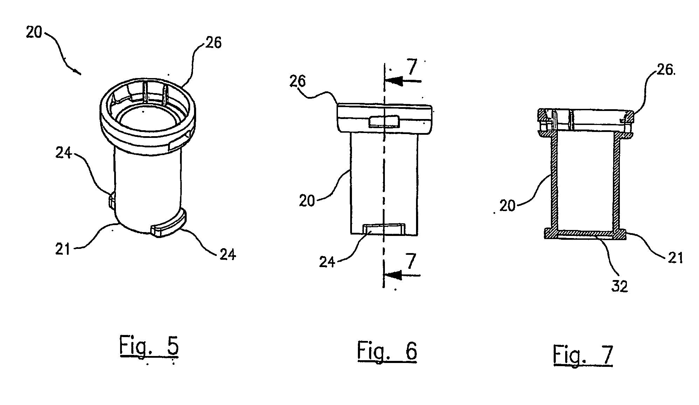

Referring initially to figures 1 to 9 of the drawings, the arrangement provides a medical instrument adapted for use as a sigmoidoscope 1. The sigmoidoscope includes a generally tubular body in the form of a speculum 2, having a forward insertion end 3, a rearward observation end 4 and a lumen 5 defining a substantially unobstructed viewing path between the observation and insertion ends.

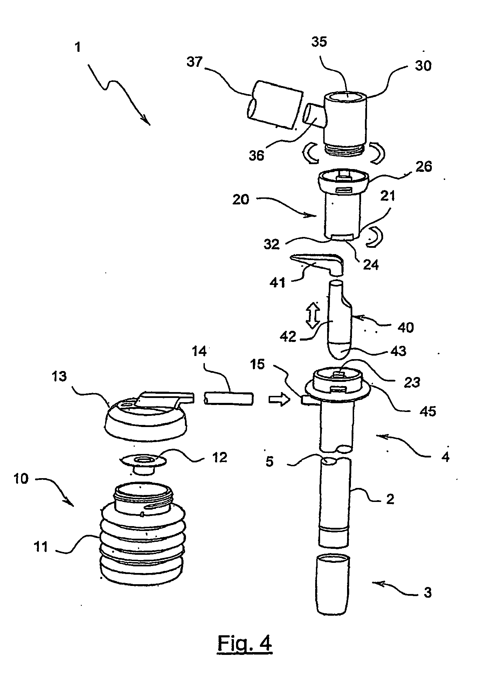

The sigmoidoscope further includes manually operable insufflation means 10 (see figure 4), including resiliently deformable bellows 11, valve mechanism 12, bellows cap 13 and a flexible connection tube 14. The connection tube extends from the bellows cap to a tubular spigot 15 extending radially outwardly from the speculum, to convey the insufflation medium, in this case air, from the bellows directly to the interior region or lumen 5 of the speculum. It will be appreciated, however, that the insufflation tube may be releasably connected to the speculum by any suitable means other than a spigot, or may be formed integrally with the speculum. Moreover, the insufflation tube itself need not be flexible, but could alternatively take the form of a rigid tube, or an internal conduit formed integrally with the speculum, the insufflation bellows or an associated housing, for example.

A generally tubular coupling element 20 includes a proximal end 21 adapted for releasable connection to the observation end 4 of the speculum. This connection takes the form of a twist-lock mechanism comprising lugs 23 extending radially inwardly from the observation end of the speculum, for twist-locking engagement with complementary lugs 24 extending radially outwardly from the proximal end of the coupling element. Importantly, this connection is adapted to provide an airtight seal between the two components by means of an O-ring or other suitable sealing elements. The coupling element 20 includes a distal end 26 adapted for threaded engagement with an optical head 30. The coupling element further includes an optically transparent observation window 32 disposed toward the proximal end in a plane generally normal to the longitudinal axis, so as effectively to isolate the distal end from the proximal end.

In this way, the coupling element is adapted upon assembly to substantially seal the observation end of the speculum and to space the optical head apart from the speculum in order to avoid direct contact between these components. At the same time, the coupling element maintains coaxial alignment between the speculum and the optical head without visually obstructing the viewing path. The coupling element is preferably between 1 cm centimetre and around 10 cm in length, and ideally between 3 cm and around 6 cm in length.

The optical head 30 incorporates an eyepiece 35, which may optionally include a magnifying lens, to facilitate visual inspection of the colon through the observation window of the coupling element and the lumen of the speculum, in a manner well known and understood by those skilled in the art. The optical head also incorporates an attachment formation 36 enabling releasable connection of a light source 37. The attachment formation 36 is oriented at an oblique angle, to avoid obstruction of the eyepiece by the light source.

The optical head further includes fibre-optic elements adapted to transmit light from the light source to the distal end of the coupling element. The coupling element and speculum are formed from translucent materials adapted to transmit light received from the optical head to the insertion end of the speculum, thereby in use to the illuminate the insufflated region of the colon.

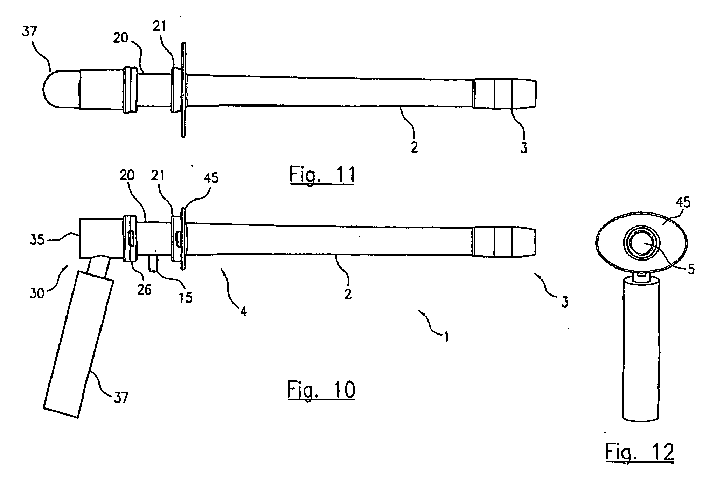

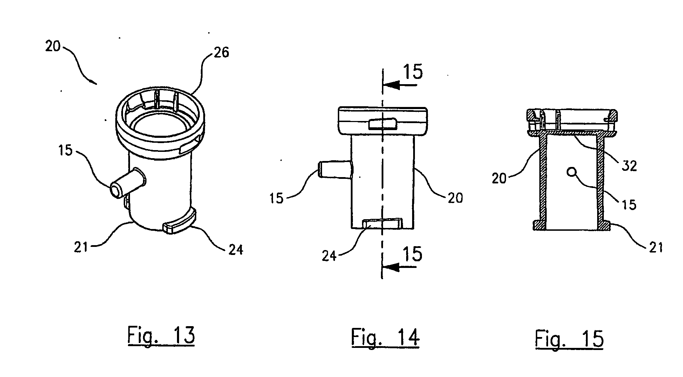

The embodiment of the invention is shown in figures 10 to 15, wherein corresponding features are denoted by corresponding reference numerals. In this embodiment, the spigot 15 extends outwardly from the coupling element 20, such that the insufflation medium is fed indirectly into the lumen 5 of the speculum 2, via the coupling element. In this case, the observation window 32 is positioned intermediate the insufflation spigot and the distal end 26 of the coupling element. Advantageously, this embodiment of the invention can be readily "retrofitted" for use in conjunction with conventional specula.

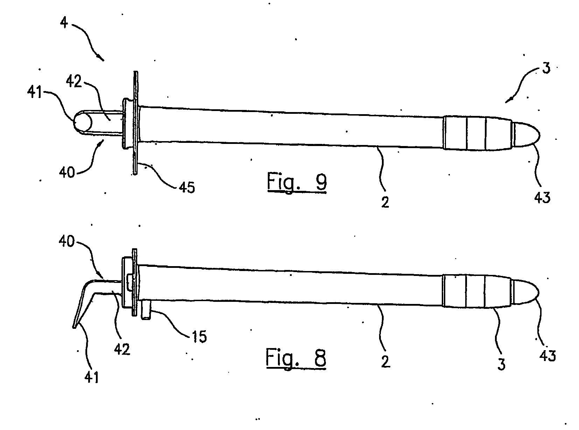

Turning now to describe the operation of the instrument, the sigmoidoscope is initially assembled to the stage shown in figures 8 and 9. This corresponds to the insertion configuration, in which the coupling element and optical head are detached, and a removable obturator 40 is fed through the lumen of the speculum. The obturator includes a handle 41, an elongate body 42, and a domed head 43. The obturator is inserted to the maximum extent, defined by the point at which a stop on the body of the obturator abuts the observation end of the speculum. At this point, the domed head protrudes smoothly and contiguously beyond the peripheral rim at the forward end 3 of the speculum. The insufflation bulb and tube are then attached via the spigot 15 on the observation end of the speculum (see figure 4).

With the instrument thus assembled, the sigmoidoscope is suitably lubricated and inserted into the bowel cavity of the patient, with the domed head on the obturator facilitating the rectal insertion procedure. During this process, a radial flange 45 on the speculum facilitates manipulation, prevents over-insertion, and shields the observation end of the speculum, as far as possible, from contamination. Once the instrument is in position, the obturator is carefully removed and discarded to provide an unobstructed axial viewing path through the lumen of the speculum.

Next, the coupling element is twist-locked onto the observation end of the speculum, so as to seal the lumen at that end, and the optical head in turn is threadedly connected to the distal end of the coupling element. Finally, the light source is attached to the optical head. This corresponds to the observation configuration of the instrument, as best seen in figures 1 and 2, and also in figures 10 and 11. The patient's bowel is then insufflated by manual squeezing of the insufflation bellows.

Finally, the light source in the optical head is energised, whereby light from the source travels through the fibre-optic elements in the optical head, through the coupling element, and along the speculum to the insertion end, from where it is emitted from the tube. This illuminates the insufflated area of the colon in the region observable by the physician looking through the eyepiece, to allow unobstructed visual inspection. During the inspection procedure, it will be appreciated that the optical head and light source are spaced apart from, and totally isolated, from the lumen, the speculum and the insufflation medium, by the coupling element.

If the insertion end of the speculum should become occluded by faecal matter at any stage during the procedure, the coupling element with the optical head and light source attached may be readily removed from the speculum as a discrete sub-assembly by a simple reverse twist-locking action between the coupling element and the speculum. This permits removal of the occlusion by re-insertion of an obturator, pledget or other suitable instrument(s). The obturator or pledget is then removed and the coupling element together with the optical head reattached. Other procedures involving the use of instruments through the speculum may be performed in like manner.

After the visual inspection has been completed, the optical head and light source are unscrewed from the coupling element, and the speculum is removed from the patient. The speculum and coupling element can then be discarded together as a disposable assembly. It is envisaged that the insufflation bellows and tube would also be disposed of with the speculum, since all of these components would be potentially contaminated as a result of direct contact with the patient, and are readily replaceable. With appropriate bacterial filtration mechanisms or sterilisation protocols, however, the insufflator in some arrangements may be reused.

The optical head and light source, which will not have come into direct contact with either the patient or the insufflation medium, are retained for use with fresh sigmoidoscopes on subsequent patients. This is important because the capital cost of these precision instruments is too high to justify disposal after each use, and the cleaning and decontamination processes that would otherwise be required using know sigmoidoscopy techniques are time-consuming and expensive.

The invention thus provides a simple, elegant, efficient and cost-effective medical instrument, particularly well adapted for use as a sigmoidoscope, in which the components susceptible to contamination are inexpensive and readily disposable, while the more costly components are effectively isolated from contamination so as to be readily reusable. Further, the invention permits initial and intermittent insertion of an obturator through the speculum by simple disconnection of the proximal end of the coupling element. At the same time, the coupling element supports the optical head and light source in spaced apart relationship from the speculum, and the at all times isolates the optical head and light source from direct contact with both the speculum and the insufflation medium. This substantially eliminates the risk of contamination of the optical head and the consequential risk of cross-contamination of subsequent patients. A further advantage in the case of the embodiment of the invention as described, is that it can be readily retrofitted for use in conjunction with conventional specula. In all these and other respects, the invention represents a practical and commercially significant improvement over the prior art.

Although the invention has been described with reference to specific examples, it will be appreciated by those skilled in the art that the invention may be embodied in many other forms.

## Claims

1. A sigmoidoscope adapted for use in conjunction with a removable obturator to facilitate internal examination of the sigmoid colon of a patient, said sigmoidoscope including:
a generally tubular speculum having a sidewall defining a forward insertion end, a rearward observation end and an internal lumen, the lumen defining a substantially unobstructed viewing path between the observation end and the insertion end; manually operable insufflation means in fluid communication with the lumen of the speculum, for insufflation of the sigmoid colon with an insufflation medium;
a coupling element having a proximal end for releasable connection to the observation end of the speculum, a distal end, and an optically transparent observation window effectively isolating the distal end from the proximal end;
the coupling element being adapted upon connection to substantially seal the observation end of the speculum,
**characterised in that** the distal end of the coupling element is adapted for releasable connection to an optical head, and being adapted upon connection to prevent direct contact between the optical head and the speculum, without visually obstructing the viewing path;
the insufflation means being adapted to establish direct fluid communication with the connected coupling element such that the insufflation medium is conveyed indirectly into the lumen of the speculum, via the coupling element.

2. A sigmoidoscope according to claim 1, adapted to permit initial and intermittent insertion of an obturator through the speculum upon disconnection of the proximal end of the coupling element, while isolating the optical head from direct contact with the speculum and the insufflation medium, so as to substantially eliminate the risk of contamination of the optical head.

3. A sigmoidoscope according to claim 1 or claim 2, wherein the insufflation means includes an insufflation tube connected by means of a hollow insufflation spigot on the coupling element.

4. A sigmoidoscope according to claim 3, wherein the observation window is positioned intermediate the insufflation spigot and the distal end of the coupling element.

5. A sigmoidoscope according to any one of the preceding claims, wherein one or more of the speculum, the insufflation means and the coupling element are disposable, and wherein the optical head is reusable.

6. A sigmoidoscope according to any one of the preceding claims, wherein the optical head includes connection means to enable releasable connection of a light source.

7. A sigmoidoscope according to claim 6, wherein the speculum and the coupling element are formed from optically transparent or translucent plastics materials adapted to conduct light from the light source to the insertion end of the speculum, for illumination of an insufflated region of the colon.

8. A sigmoidoscope according to any one of the preceding claims, wherein the proximal end of the coupling element is adapted for releasable connection with the speculum by means of a first connection mechanism and associated sealing surfaces arranged such that in use the connecting element effectively seals the lumen at the observation end of the speculum.

9. A sigmoidoscope according to claim 8, wherein the first connection mechanism is selected from the group comprising a twist-lock connection, a threaded connection, a hinged connection, a tapered connection, a sliding connection, a magnetic connection or a press-fitted connection.

10. A sigmoidoscope according to claim 8 or claim 9, wherein the first connection mechanism takes the form of a first twist-lock connector and the associated sealing surfaces are adapted to prevent passage of any contaminated insufflation medium around or through the observation window to the distal end of the connecting element.

11. A sigmoidoscope according to claim 10, wherein the first twist-lock connector comprises a plurality of lugs extending radially inwardly from the observation end of the speculum, for twist-locking engagement with complementary lugs extending radially outwardly from the proximal end of the coupling element.

12. A sigmoidoscope according to any one of the preceding claims, wherein the distal end of the coupling element is adapted for releasable connection to the optical head by means of a second connection mechanism.

13. A sigmoidoscope according to claim 12, wherein the second connection mechanism is selected from the group comprising a twist-lock connection, a threaded connection, a hinged connection, a tapered connection, a sliding connection, a magnetic connection or a press-fitted connection.

14. A sigmoidoscope according to claim 12 or claim 13, wherein the second connection mechanism comprises a threaded connection.

15. A sigmoidoscope according to any one of the preceding claims, wherein the observation window is disposed in a plane generally normal to a longitudinal axis of the coupling element, so as effectively to isolate the distal end from the proximal end.

16. A sigmoidoscope according to any one of the preceding claims, wherein the coupling element is adapted to maintain relative coaxial alignment between the speculum and the optical head, and wherein the optical head provides for oblique connection of the light source, thereby to provide an unobstructed axial viewing path through the optical head, the coupling element and the lumen.

17. A sigmoidoscope according to any one of the preceding claims, wherein the coupling element is between 1 cm (centimetre) and around 10 cm in length.

18. A sigmoidoscope according to any one of the preceding claims, wherein the coupling element is between 3 cm and around 6 cm in length.

19. A sigmoidoscope according to any one of the preceding claims, wherein disconnection of the coupling element with the optical head attached permits selective insertion and withdrawal of an obturator through the speculum, without direct contact between the obturator or the insufflation medium and any part of the optical head or the light source.

20. A coupling element configured for use in conjunction with conventional sigmoidoscope specula, said coupling element including:
a proximal end for releasable connection to the speculum, a distal end, and an optically transparent observation window effectively isolating the distal end from the proximal end,
**characterised in that** the distal end of the coupling element is adapted for releasable connection to an optical head,
wherein the coupling element may be releasably connected to insufflation means, such that an insufflation medium can be fed indirectly from the insufflation means into the speculum, via the coupling element,
the observation window being positioned such that during use it is intermediate the connected insufflation means and the distal end of the coupling element.

21. A coupling element according to claim 20, wherein the insufflation means includes an insufflation tube connected by means of a hollow insufflation spigot on the coupling element.

## Patentansprüche

1. Sigmoidoskop, das zur Verwendung in Verbindung mit einem abnehmbaren Obturator geeignet ist, um eine innere Untersuchung des Sigmoideumabschnitt des Dickdarms zu erleichtern, wobei das Sigmoidoskop Folgendes umfasst:
ein im Allgemeinen röhrenförmiges Spekulum mit einer Seitenwand, die ein vorderes Einführungsende, ein hinteres Betrachtungsende und ein Lumen im Inneren definiert, wobei das Lumen einen im Wesentlichen freien Sichtweg zwischen dem Betrachtungsende und dem Einführungsende definiert; manuell bedienbare Insufflationsmittel, die sich in Fluidkommunikation mit dem Lumen des Spekulums befinden, zur Insufflation des Sigmoideumabschnitts des Dickdarms mit einem Insufflationsmedium;
ein Verbindungselement mit einem proximalen Ende zur lösbaren Verbindung mit dem Betrachtungsende des Spekulums, einem distalen Ende und einem optisch transparenten Betrachtungsfenster, das das distale Ende effektiv von dem proximalen Ende trennt;
wobei das Verbindungselement geeignet ist, um bei Verbindung das Betrachtungsende des Spekulums im Wesentlichen abzudichten,
**dadurch gekennzeichnet, dass** das distale Ende des Verbindungselements geeignet ist, um lösbar mit einem optischen Kopf verbunden zu werden, und geeignet ist, um bei Verbindung einen direkten Kontakt zwischen dem optischen Kopf und dem Spekulum zu verhindern, ohne die Sicht durch den Sichtweg zu versperren;
wobei das Insufflationsmittel geeignet ist, um eine direkte Fluidkommunikation mit dem verbundenen Verbindungselement herzustellen, so dass das Insufflationsmedium indirekt über das Verbindungselement in das Lumen des Spekulums transportiert wird.

2. Sigmoidoskop nach Anspruch 1, das geeignet ist, um, wenn das proximale Ende des Verbindungselements nicht verbunden ist, ein Einführen eines Obturators zu Beginn oder intermittierend durch das Spekulum zu ermöglichen, während der optische Kopf in Bezug auf direkten Kontakt mit dem Spekulum und dem Insufflationsmedium isoliert wird, um das Risiko einer Verschmutzung des optischen Kopfes im Wesentlichen zu eliminieren.

3. Sigmoidoskop nach Anspruch 1 oder 2, worin das Insufflationsmittel ein Insufflationsröhrchen umfasst, das über einen hohlen Insufflationszapfen an das Verbindungselement angeschlossen ist.

4. Sigmoidoskop nach Anspruch 3, worin das Betrachtungsfenster zwischen dem Insufflationszapfen und dem distalen Ende des Verbindungselements angeordnet ist.

5. Sigmoidoskop nach einem der vorangegangenen Ansprüche, worin ein oder mehrere Elemente von Spekulum, Insufflationsmittel und Verbindungsmittel zum einmaligen Gebrauch dienen und worin der optische Kopf wiederverwendbar ist.

6. Sigmoidoskop nach einem der vorangegangenen Ansprüche, worin der optische Kopf Verbindungsmittel umfasst, um das lösbare Anschließen einer Lichtquelle zu ermöglichen.

7. Sigmoidoskop nach Anspruch 6, worin das Spekulum und das Verbindungselement aus optisch transparenten oder transluzenten Kunststoffmaterialien bestehen, die geeignet sind, um Licht von der Lichtquelle durch das Einführungsende des Spekulums zu leiten, um einen insufflierten Bereich des Dickdarms auszuleuchten.

8. Sigmoidoskop nach einem der vorangegangenen Ansprüche, worin das proximale Ende des Verbindungselements geeignet ist, um durch einen ersten Verbindungsmechanismus und damit zusammenhängende Dichtungsflächen, die so angeordnet sind, dass das Verbindungselement bei Verwendung das Lumen am Betrachtungsende des Spekulums wirksam abdichtet, eine lösbare Verbindung mit dem Spekulum herzustellen.

9. Sigmoidoskop nach Anspruch 8, worin der erste Verbindungsmechanismus aus der aus folgenden bestehenden Gruppe ausgewählt ist: einer Twist-Lock-Verbindung, Schraubverbindung, Scharnierverbindung, Konusverbindung, Schiebverbindung, Magnetverbindung oder Einpressverbindung.

10. Sigmoidoskop nach Anspruch 8 oder 9, worin der erste Verbindungsmechanismus in Form einer ersten Twist-Lock-Verbindung vorliegt und die damit zusammenhängenden Dichtungsflächen geeignet sind, um zu verhindern, dass verunreinigtes Insufflationsmedium um oder durch das Betrachtungsfenster zu dem distalen Ende des Verbindungselements gelangt.

11. Sigmoidoskop nach Anspruch 10, worin die erste Twist-Lock-Verbindung eine Vielzahl an Ansätzen umfasst, die sich von dem Betrachtungsende des Spekulums aus radial nach innen erstrecken, um mit komplementären Ansätzen, die sich von dem proximalen Ende des Verbindungselements radial nach außen erstrecken, in Twist-Lock-Eingriff zu gelangen.

12. Sigmoidoskop nach einem der vorangegangenen Ansprüche, worin das distale Ende des Verbindungselements geeignet ist, um durch einen zweiten Verbindungsmechanismus lösbar mit dem optischen Kopf verbunden zu werden.

13. Sigmoidoskop nach Anspruch 12, worin der zweite Verbindungsmechanismus aus der aus folgenden bestehenden Gruppe ausgewählt ist: Twist-Lock-Verbindung, Schraubverbindung, Scharnierverbindung, Konusverbindung, Schiebverbindung, Magnetverbindung oder Einpressverbindung.

14. Sigmoidoskop nach Anspruch 12 oder 13, worin der zweite Verbindungsmechanismus eine Schraubverbindung umfasst.

15. Sigmoidoskop nach einem der vorangegangenen Ansprüche, worin das Betrachtungsfenster in einer Ebene angeordnet ist, die im Allgemeinen normal auf die Längsachse des Verbindungselements steht, um das distale Ende in Bezug auf das proximale Ende wirksam zu isolieren.

16. Sigmoidoskop nach einem der vorangegangenen Ansprüche, worin das Verbindungselement geeignet ist, um eine relative koaxiale Ausrichtung des Spekulums und des optischen Kopfes aufrecht zu erhalten, und worin der optische Kopf eine Schrägverbindung mit der Lichtquelle bereitstellt, um **dadurch** einen freien axialen Sichtweg durch den optischen Kopf, das Verbindungselement und das Lumen bereitzustellen.

17. Sigmoidoskop nach einem der vorangegangenen Ansprüche, worin das Verbindungselement zwischen 1 cm (Zentimeter) und etwa 10 cm lang ist.

18. Sigmoidoskop nach einem der vorangegangenen Ansprüche, worin das Verbindungselement zwischen 3 cm und etwa 6 cm lang ist.

19. Sigmoidoskop nach einem der vorangegangenen Ansprüche, worin ein Abnehmen des Verbindungselements mit dem daran befestigten optischen Kopf ein selektives Einführen und Zurückziehen eines Obturators durch das Spekulum ermöglicht, ohne dass es zu einem direkten Kontakt zwischen dem Obturator oder dem Insufflationsmedium und irgendeinem Teil des optischen Kopfes oder der Lichtquelle kommt.

20. Verbindungselement, das geeignet ist, um in Verbindung mit herkömmlichen Spekula für Sigmoidoskope verwendet zu werden, wobei das Verbindungselement Folgendes umfasst:
ein proximales Ende um lösbar mit dem Spekulum verbunden zu werden, ein distales Ende und ein optisch transparentes Betrachtungsfenster, das das distale Ende wirksam in Bezug auf das proximale Ende trennt, **dadurch gekennzeichnet, dass** das distale Ende des Verbindungselements geeignet ist, um lösbar mit einem optischen Kopf verbunden zu werden,
worin das Verbindungselement lösbar mit einem Insufflationsmittel verbunden werden kann, so dass Insufflationsmedium indirekt von dem Insufflationsmittel über das Verbindungselement in das Spekulum zugeführt werden kann,
wobei das Betrachtungsfenster so angeordnet ist, dass es während der Verwendung zwischen dem angeschlossenen Insufflationsmittel und dem distalen Ende des Verbindungselements vorliegt.

21. Verbindungselement nach Anspruch 20, worin das Insufflationsmedium ein Insufflationsröhrchen umfasst, das über einen hohlen Insufflationszapfen an das Verbindungselement angeschlossen ist.

## Revendications

1. Sigmoidoscope apte à être utilisé conjointement avec un obturateur amovible pour faciliter l'examen interne du côlon sigmoïde d'un patient, ledit sigmoïdoscope comprenant:
un speculum généralement tubulaire ayant une paroi latérale définissant une extrémité d'insertion avant, une extrémité d'observation arrière et une lumière interne, la lumière définissant un chemin de vision sensiblement non obstruit entre l'extrémité d'observation et l'extrémité d'insertion; un moyen d'insufflation actionnable manuellement en communication fluidique avec la lumière du speculum, pour insuffler dans le côlon sigmoïde un milieu d'insufflation;
un élément de couplage ayant une extrémité proximale pour la connection amovible à l'extrémité d'observation du speculum, une extrémité distale et une fenêtre d'observation optiquement transparente isolant efficacement l'extrémité distale de l'extrémité proximale;
l'élément de couplage étant apte, lors de la connection, à sceller sensiblement l'extrémité d'observation du speculum,
**caractérisé en ce que** l'extrémité distale de l'élément de couplage est conçue pour la connection amovible à une tête optique et étant conçue, lors de la connection, pour empêcher un contact direct entre la tête optique et le speculum, sans obstruire visuellement le chemin de vision;
le moyen d'insufflation étant apte à établir une communication fluidique directe avec l'élément de couplage connecté de telle sorte que le milieu d'insufflation est convoyé indirectement dans la lumière du speculum, par l'élément de couplage.

2. Sigmoïdoscope selon la revendication 1, apte à permettre une insertion initiale et intermittente d'un obturateur à travers le speculum lors de la déconnection de l'extrémité proximale de l'élément de couplage tout en isolant la tête optique d'un contact direct avec le speculum et le milieu d'insufflation de manière à éliminer sensiblement le risque de contamination de la tête optique.

3. Sigmoïdoscope selon la revendication 1 ou la revendication 2, dans lequel le moyen d'insufflation comporte un tube d'insufflation relié au moyen d'un bout d'insufflation creux à l'élément de couplage.

4. Sigmoïdoscope selon la revendication 3, dans lequel la fenêtre d'observation est positionnée entre le bout d'insufflation et l'extrémité distale de l'élément de couplage.

5. Sigmoïdoscope selon l'une quelconque des revendications précédentes, dans lequel un ou plusieurs parmi le speculum, le moyen d'insufflation et l'élément de couplage sont jetables, et où la tête optique est réutilisable.

6. Sigmoïdoscope selon l'une quelconque des revendications précédentes, dans lequel la tête optique comporte un moyen de connection pour permettre la connection amovible d'une source de lumière.

7. Sigmoïdoscope selon la revendication 6, dans lequel le speculum et l'élément de couplage sont réalisés à partir de matériaux plastiques optiquement transparents ou translucides aptes à conduire la lumière de la source de lumière à l'extrémité d'insertion du speculum, pour illuminer une région insufflée du côlon.

8. Sigmoïdoscope selon l'une quelconque des revendications précédentes, dans lequel l'extrémité proximale de l'élément de couplage est conçue pour une connection amovible avec le speculum au moyen d'un premier mécanisme de connection et de surfaces de scellement associées agencées de telle sorte qu'en cours d'utilisation, l'élément de connection scelle efficacement la lumière à l'extrémité d'observation du speculum.

9. Sigmoïdoscope selon la revendication 8, dans lequel le premier mécanisme de connection est sélectionné dans le groupe comprenant une connection à verrouillage rotatif, une connection filetée, une connection à charnière, une connection conique, une connection de coulissement, une connection magnétique ou une connection d'ajustement serré.

10. Sigmoïdoscope selon la revendication 8 ou la revendication 9, dans lequel le premier mécanisme de connection prend la forme d'un premier connecteur à verrouillage rotatif, et les surfaces de scellement associées sont aptes à empêcher le passage d'un milieu d'insufflation contaminé autour ou à travers la fenêtre d'observation à l'extrémité distale de l'élément de connection.

11. Sigmoïdoscope selon la revendication 10, dans lequel le premier connecteur à verrouillage rotatif comprend plusieurs pattes s'étendant radialement vers l'intérieur depuis l'extrémité d'observation du speculum, pour une mise en prise par verrouillage rotatif avec des pattes complémentaires s'étendant radialement vers l'extérieur depuis l'extrémité proximale de l'élément de couplage.

12. Sigmoïdoscope selon l'une quelconque des revendications précédentes, dans lequel l'extrémité distale de l'élément de couplage est apte à être reliée amoviblement à la tête optique au moyen d'un deuxième mécanisme de connection.

13. Sigmoïdoscope selon la revendication 12, dans lequel le deuxième mécanisme de connection est sélectionné dans le groupe comprenant une connection à verrouillage rotatif, une connection filetée, une connection à charnière, une connection conique, une connection de coulissement, une connection magnétique ou une connection d'ajustement serré.

14. Sigmoïdoscope selon la revendication 12 ou la revendication 13, dans lequel le deuxième mécanisme de connection comprend une connection filetée.

15. Sigmoïdoscope selon l'une quelconque des revendications précédentes, dans lequel la fenêtre d'observation est disposée dans un plan généralement perpendiculaire à un axe longitudinal de l'élément de couplage de manière à isoler efficacement l'extrémité distale de l'extrémité proximale.

16. Sigmoïdoscope selon l'une quelconque des revendications précédentes, dans lequel l'élément de couplage est apte à maintenir un alignement coaxial rotatif entre le speculum et la tête optique, et où la tête optique réalise une connection oblique de la source de lumière en réalisant ainsi un chemin de vision axial non obstruit à travers la tête optique, l'élément de couplage et la lumière.

17. Sigmoïdoscope selon l'une quelconque des revendications précédentes, dans lequel l'élément de couplage a une longueur entre 1 cm (centimètre) et environ 10 cm.

18. Sigmoïdoscope selon l'une quelconque des revendications précédentes, dans lequel l'élément de couplage a une longueur entre 3 cm et environ 6 cm.

19. Sigmoïdoscope selon l'une quelconque des revendications précédentes, dans lequel la déconnection de l'élément de couplage avec la tête optique attachée permet une insertion et retrait sélectifs d'un obturateur à travers le speculum, sans contact direct entre l'obturateur ou le milieu d'insufflation et n'importe quelle partie de la tête optique ou de la source de lumière.

20. Elément de couplage configuré pour utilisation conjointement avec des specula de sigmoïdoscope classiques, ledit élément de couplage comprenant:
une extrémité proximale pour la connection amovible au speculum, une extrémité distale et une fenêtre d'observation optiquement transparente isolant efficacement l'extrémité distale de l'extrémité proximale, **caractérisé en ce que** l'extrémité distale de l'élément de couplage est conçue pour une connection amovible à une tête optique, où l'élément de couplage peut être relié amoviblement au moyen d'insufflation de sorte qu'un milieu d'insufflation peut être amenée indirectement du moyen d'insufflation dans le speculum, par l'élément de couplage,
la fenêtre d'observation étant positionnée de telle sorte que durant l'utilisation, elle se trouve entre le moyen d'insufflation connecté et l'extrémité distale de l'élément de couplage.

21. Elément de couplage selon la revendication 20, dans lequel le moyen d'insufflation comporte un tube d'insufflation connecté au moyen d'un bout d'insufflation creux à l'élément de couplage.
